(19) Europäisches Patentamt
European Patent Office
Office européen des brevets

(11) EP 2 114 362 B1

(12) EUROPEAN PATENT SPECIFICATION

(45) Date of publication and mention of the grant of the patent: **18.07.2018 Bulletin 2018/29**

(21) Application number: **08723115.5**

(22) Date of filing: **25.02.2008**

(51) Int Cl.:
***A61K 8/49*** *(2006.01)* ***A61Q 19/02*** *(2006.01)*
***A61K 8/60*** *(2006.01)*

(86) International application number: **PCT/KR2008/001077**

(87) International publication number: **WO 2008/105605 (04.09.2008 Gazette 2008/36)**

# (54) COSMETIC COMPOSITION FOR SKIN WHITENING COMPRISING A MIXTURE OF ARCTIIN AND ARCTIGENIN

KOSMETISCHE ZUSAMMENSETZUNG ZUR HAUTBLEICHUNG ENTHALTEND EINE MISCHUNG AUS ARCTIIN UND ARCTIGENIN

COMPOSITION COSMÉTIQUE POUR LE BLANCHIMENT DE LA PEAU COMPRENANT UN MELANGE D'ARCTIINE ET D'ARCTIGÉNINE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **28.02.2007 KR 20070020603**

(43) Date of publication of application: **11.11.2009 Bulletin 2009/46**

(73) Proprietor: **Coreana Cosmetics Co., Ltd.**
**330-833 Cheonan-shi,**
**Chungcheongnam-do (KR)**

(72) Inventors:
- **Lee, Jung Noh**
  **Chungcheongnam-do 339-752 (KR)**
- **Choi, Jeong Eun**
  **Cheonan si**
  **Chungcheongnam-do 330-818 (KR)**
- **Kim, Sang Woo**
  **Cheonan-shi**
  **Chungcheongnam-do 330-833 (KR)**
- **Lee, Kang Tae**
  **Cheonan-si**
  **Chungcheongnam-do 330-835 (KR)**
- **Lee, Kun Kook**
  **Seoul 134-777 (KR)**

(74) Representative: **Jacobacci Coralis Harle**
**32, rue de l'Arcade**
**75008 Paris (FR)**

(56) References cited:
**DE-A1- 10 305 965** **KR-A- 20020 080 660**
**KR-A- 20020 080 660** **US-A1- 2006 093 633**

- **DATABASE WPI Week 199408 Thomson Scientific, London, GB; AN 1994-061970 XP002715811, & JP H06 16525 A (KANEBO LTD) 25 January 1994 (1994-01-25)**
- **DATABASE WPI Week 200710 Thomson Scientific, London, GB; AN 2007-097391 XP002715812, & KR 100 599 491 B1 (UNIV SEOUL NAT IND FOUND) 12 July 2006 (2006-07-12)**
- **DATABASE WPI Week 200376 Thomson Scientific, London, GB; AN 2003-806560 XP002715813, & JP 2002 201122 A (MARUZEN SEIYAKU KK) 16 July 2002 (2002-07-16)**
- **DATABASE WPI Week 200281 Thomson Scientific, London, GB; AN 2002-748665 XP002715814, & KR 2002 0046614 A (KOREAN COSMETICS CO LTD) 21 June 2002 (2002-06-21)**
- **DATABASE WPI Week 199551 Thomson Scientific, London, GB; AN 1995-400876 XP002715815, & JP H07 277944 A (NARISU KESHOHIN KK) 24 October 1995 (1995-10-24)**
- **DATABASE WPI Week 200377 Thomson Scientific, London, GB; AN 2003-820950 XP002715816, & JP 2003 246722 A (KANEBO LTD) 2 September 2003 (2003-09-02)**
- **DATABASE WPI Week 200355 Thomson Scientific, London, GB; AN 2003-580926 XP002715817, & JP 2003 081748 A (POLA CHEM IND INC) 19 March 2003 (2003-03-19)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- DATABASE WPI Week 200035 Thomson Scientific, London, GB; AN 2000-402930 XP002715818, & JP 2000 119156 A (KOSE KK) 25 April 2000 (2000-04-25)

## Description

### Technical Field

The present invention relates to the use of a cosmetic composition for skin whitening comprising a mixture of arctiin and arctigenin as an active ingredient.

### Background Art

Everyone consistently wants to have white and fine skin. In general, the color of skin, hair, eyes, etc. of a human depends on melanin, carotin, hemoglobin, etc. Especially, melanin also is the primary determinant of human skin color. In other words, skin color depends on the amount, property, and distribution of the melanin. A pigment in skin or hair of a human plays a role of protecting the skin or hair from a harmful affect of sunlight, especially ultraviolet rays. For example, a human lacking such a pigment is very sensitive to sunlight, and is subject to a burn, and thus even if he/she is young, there is a high probability of getting skin cancer. Ultraviolet rays of short wavelength (290-320 nm) and carcinogen form a harmful radical, for example, an oxygen radical, in skin, and thus, such an oxygen radical inducing the skin aging by attacking the skin cells.

Pigmentation by ultraviolet rays may be directly caused by DNA damage. Also, when cytokine, a growth factor, and a hormone secreted by the action of ultraviolet rays on keratinocytes and fibroblast stimulate a melanin cell, such pigmentation may be caused. The keratinocytes secrete $\alpha$-melanocyte stimulating hormone (a-MSH), agouti signaling protein (ASP), endothelin, etc. by inflammation and irradiation of ultraviolet rays, thereby promoting the synthesis of melanin. $\alpha$-MSH is mainly secreted from the anterior pituitary gland, and also is secreted from keratinocytes by inflammation or irradiation of ultraviolet rays. $\alpha$-MSH is generated by protein hydrolysis of POMC (Proopiomelanocortin) protein secerted from keratinocytes stimulated by ultraviolet rays, and inoculation of $\alpha$-MSH into the cultured melanin cells increases the growth of the melanin cells and the activity of tyrosinase.

A main function of melanin is to remove such a harmful radical, and thus to protect skin from damage caused by the radical. Accordingly, a large amount of melanin indicates that there is an effective system for protecting skin from physical/chemical toxic substances.

Meanwhile, in the synthesis of melanin, tyrosine is converted into DOPA and then into dopaquinone by enzyme activity of tyrosinase, and then, nonenzymatic oxidation is performed to synthesize the melanin. Then, through a cycle process, the synthesized melanin is transferred to a skin cell, and then, is lost by epidermolysis.

Such melanin synthesis process, which is a natural phenomenon, does not excessively occur in human skin in a normal state, but excessively occurs in skin stimulated by external stimuli such as ultraviolet rays, environmental pollution, stress, etc. When such excessively synthesized melanin remains in skin without being discharged to the outside of the skin, pigmentation occurs, thereby generating melasma, freckles, etc.

Therefore, skin whitening may be achieved by inhibiting the synthesis of melanin, and representative methods of inhibiting the synthesis of melanin include blocking of an external stimulus such as ultraviolet rays, inhibition of activation of tyrosinase related to melanin synthesis, inhibition of division of a melanin cell related to melanin synthesis, use of a vitamin C derivative, etc.

In a conventional whitening cosmetic used for skin-whitening or amelioration of melasma and freckles, a substance having inhibitory activity to tyrosinase, such as hydroquinone, ascorbic acid, kojic acid, glutathione, etc. is blended with ointment or a cosmetic. However, hydroquinone has a skin-whitening effect, but has a limitation in the usage amount due to high skin irritation, and because ascorbic acid easily is oxidated, a cosmetic including the ascorbic acid is subject to color-change or deterioration. Also, a thiol-based compound such as glutathione, cysteine, etc. is not appropriate as a blending component of a cosmetic because the compound has peculiar unpleasant smells, and poor transdermal absorption property, and its glycoside and derivatives have high polarity.

Recently, in order to solve the above-described problems, much research and development have been carried out by many cosmetic companies, and accordingly, it has been found that various natural substances, such as cotex of *Morus alba*, *Glycyrrhiza uralensis*, *Dendropanax*, *Asparagus cochinchinensis*, *Ilex rotunda*, *Forsythia suspensa* , *Arctium lappa fruit*, etc. are effective in skin whitening. However, most of the substances are extracts in which various ingredients are mixed, and thus, the use of such substances with high concentration may lower the stability of a cosmetic, irritate the skin, and relatively decrease the whitening effect according to the concentration.

### Disclosure of Invention

### Technical Problem

Therefore, the inventors of the present invention have tried to develop a substance for controlling and inhibiting

the synthesis of melanin by acting on a melanin synthesis route of α-MSH, and to solve problems of a conventional skin whitening cosmetic composition, such as stability, sensitiveness, a relatively weak whitening effect of a cosmetic. In result, it has been found that a composition including arctiin and arctigenin show a high whitening effect and skin stability.

Accordingly, one object of the present invention is to provide the use of a cosmetic composition for skin whitening comprising a mixture of arctiin and arctigenin as an active ingredient.

**Technical Solution**

Arctigenin used as an active ingredient of the cosmetic composition according to the present invention is a ligand compound having a molecular weight of 372, 4-[(3,4-Dimethoxyphenyl)methyl]dihydro-3-[(4-hydroxy-3-metoxyphenyl)methyl]-2(3 H)-furanone, which is represented by Chemical Formula 1. Arctiin, which is glycoside of the arctigenin and includes glucose attached thereon (Formula 1), is a ligand compound having a molecular weight of 535, 4-[(3,4-Dimethoxyphenyl)methyl] dihydro-3-[(4-Glucose-3-metoxyphenyl)methyl]-2(3H)-furanone. Both the arctiin and the arctigenin are ligand compounds, and include very high whitening activity compared to various ingredients included in forsythia suspensa, such as matairesinol, caffeoyl glycoside, forsythoside, oleanolic acid, betulinic acid, rutin, and ursolic acid (refer to Examples 1 and 3). The arctiin and the arctigenin of the present invention may be extracted from forsythia suspensa (forsythia koreana), and is commercially available. Also, the arctiin and the arctigenin of the present invention may be extracted from *burdock*(*arctium lappa*), burdock's dried fruit, *safflower*(*carthamus tinctorius*), etc.

ChemistryFigure 1

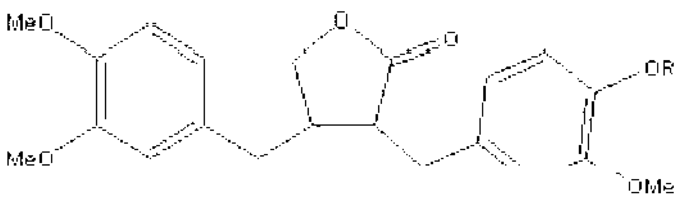

R=H Arctigenin
R=Glc Arctiin

The term "ligand compound" of the present invention indicates a diphenol compound, which is called phytoestrogen due to its similar structure and molecular weight to steroid estrogen. Also, the ligand compound is effective in hormone dependent diseases due to its similar action to steroid estrogen. It is known that the ligand compound reduces diseases such as breast cancer, large intestine cancer, and prostatic carcinoma, inhibits heart disease and osteoporosis in postmenopausal women, and has antioxidative/anti-inflammatory/antivirus effects (The Royal Soc. of Chemistry, UK, 1996;Anderson JJB, Garner SC, Nutr. Res.,17:1617-1632,1997).

The arctiin and the arctigenin used in the present invention can control or inhibit the synthesis of melanin by acting on a melanin synthesis route of α-MSH secreted by UV, thereby whitening skin color.

The arctiin and the arctigenin of the present invention may be extracted from natural sources such as *burdock*(*arctium lappa*), burdock's dried fruit, safflower( *carthamus tinctorius*), forsythia suspensa, etc., and may be chemically synthesized.

In the mixture of arctiin and arctigenin in the present invention, the arctiin and the arctigenin are mixed in a weight ratio of 3:1 to 1:3.

The content of arctiin and arctigenin in the present invention is of 0.001 to 15.0 wt%, and preferably of 0.1 to 8.0 wt%, based on the total weight of the cosmetic composition.

If the content of arctiin, arctigenin or the mixture thereof in the present invention is less than 0.00001 wt%, a significant whitening effect is not achieved. On the other hand, if the content of arctiin and arctigenin is more than 40.0wt%, a whitening effect corresponding to the increased content is not significantly achieved, and problems related to skin irritation and stability of formulation may occur.

The cosmetic composition of the present invention further includes components which are conventionally used in cosmetic compositions, for example, conventional additives, such as an antioxidant, a stabilizer, a solubilizing agent, vitamins, a pigment and perfume, and carrier components.

The cosmetic composition according to the present invention may be prepared into any conventional formulations known in the art, and the formulations may include, but are not limited to, solution, suspension, emulsion, paste, gel, cream, lotion, powder, soap, surfactant containing cleaning, oil, powder foundation, emulsion foundation, wax foundation, spray, etc. More specifically, the cosmetic composition may be prepared into formulations such as skin softner(skin

lotion), nutrition lotion, nutrition cream, massage cream, essence, eye cream, cleansing cream, cleansing foam, cleansing water, pack, spray, powder, etc.

When the formulation of the cosmetic composition according to the present invention is paste, cream, or gel, as a carrier ingredient, animal oil, vegetable oil, wax, paraffin, starch, tragacanth, a cellulose derivative, polyethylene glycol, silicon, bentonite, silica, talc, zinc oxide, etc. may be used.

When the formulation of the cosmetic composition according to the present invention is powder or spray, as a carrier ingredient, lactose, talc, silica, aluminum hydroxide, calcium silicate, or polyamide powder may be used, and especially, the spray formulation may further include a propellant, such as chloro fluoro hydrocarbon, propane/butane, or dimethyl ether.

When the formulation of the cosmetic composition according to the present invention is solution or emulsion, as a carrier ingredient, a solvent, a solubilizer or an emulsifier (for example, water, ethanol, isopropanol, ethyl carbonate, ethyl acetate, benzyl alcohol, benzyl benzoate, propylene glycol, 1,3-butylglycol oil, glycerol aliphatic ester, polyethylene glycol, or sorbitan fatty acid ester) may be used.

When the formulation of the cosmetic composition according to the present invention is suspension, as a carrier ingredient, a liquid diluent (such as water, ethanol, or propylene glycol), a suspension (such as ethoxylated isostearyl alcohol, polyoxyethylene sorbitol ester, polyoxyethylene sorbitan ester), microcrystalline cellulose, aluminum metahydroxide, bentonite, agar, or tragacanth, etc. may be used.

When the formulation of the cosmetic composition according to the present invention is a surfactant containing cleansing, as a carrier ingredient, aliphatic alcohol sulfate, aliphatic alcohol ether sulfate, sulfosuccinic acid monoester, isethionate, an imidazolinium derivative, methyl taurate, sarcosinate, fatty acid amide ether sulfate, alkyl amido betaine, aliphatic alcohol, fatty acid glyceride, fatty acid diethanolamide, vegetable oil, lanolin derivative, ethoxylated glycerol fatty acid ester, etc. may be used.

Reference will now be made in detail to the preferred embodiments of the present invention. However, the following examples are illustrative only, and the scope of the present invention is not limited thereto.

## Preparation Example 1: Preparation of active substances from forsythia suspensa

To 1kg of Forsythia suspensa (the fruit of *Forsythia Koreana*) powder, which was washed with purified water, dried, and powdered, 5L of 95% ethanol was added, and reflux-agitation was carried out. Then, through filtration and concentration, 33.2g of concentrate was obtained. Next, n-hexane was added, and a degreasing process (fraction and separation) was carried out to obtain 19.3g of concentrate. The concentrate was separated by using cation exchange resin Diaion HP-20(column 25X100 cm) as water and methanol were added, the concentration of methanol being sequentially elevated (10% methanol, 20% methanol, etc.). From among the fractions, a 60~80% methanol fraction was concentrated to obtain 3.5g of concentrate, and the concentrate was dissolved in methanol. Through separation of LH-20 and MPLC, 0.63g of arctiin was obtained, which was observed by NMR, Mass, etc. Other active substances of forsythia suspensa, such as arctigenin, matairesinol, caffeoyl glycoside, forsythiaside, oleanolic acid, betulinic acid, rutin, and ursolic acid, were purchased from Sigma or Fluka. Each of the active substances,such as arctiin and arctigenin, was independently dissolved in 1,3-butylene glycol at a high concentration (1,000ppm), and was either diluted or not diluted.

## Preparation Example 2: Preparation of mixture of arctiin and arctigenin

Arctiin, which was extracted from Forsythia suspensa and prepared in the same manner as described in Preparation Example 1, was mixed with arctigenin purchased from Sigma in a weight ratio of 1:3 to 3:1, and was dissolved in 1,3-butylene glycol at a high concentration (4,000ppm).

## Experimental Example 1: Melanin synthesis inhibition bv forsythia suspensa active substances

$\alpha$-MSH, for an experiment for melanin synthesis inhibition, was dissolved in 10% DMSO(Dimethyl sulfoxide), and was used as ImM solution. B-16 melanoma (ATCC CRL 6323) cell line (derived from mice) was inoculated into a DMEM medium including 4.5 g/L of glucose, 10% serum, and 1% antibiotics, and was subcultured in 75□ T-flask under 5% $CO_2$ and at 37°C. The subcultured melanoma cell line was cultured in 75□ T-flask for 24 hours, and was treated with 0.05% Trypsin containing 0.02% EDTA. Then, cells were separated, were inoculated in □75 T-flask, and were further cultured for 6 hours. Herein, the number of cells was $1 \times 10^7$ cells/flask. Then, a melanocyte-stimulating hormone was diluted in a DMEM medium to a final concentration 100□/□. Next, each of arctiin, arctigenin, matairesinol, oleanolic acid, betulinic acid, rutin, and ursolic acid was diluted in the DMEM medium to a final concentration of 10 - 1000□/□ to carry out treatment of melanoma cells, and then, the cells were further cultured at 37°C for 5 days. Next, the medium was obtained to measure absorbance at 475nm, and through comparison of melanin synthesis, $IC_{50}$(□/□), that is, the

density for inhibiting melanin synthesis by 50%, was calculated. Table 1 shows the results.

Table 1

| Melanin synthesis inhibition ($IC_{50}$(□/□)) | | |
|---|---|---|
| Tested substances | $IC_{50}$ (□/□) | Company |
| Arctiin | **<40** | Prepared |
| Arctigenin | **<40** | Sigma |
| Matairesinol | <200 | fluka |
| Oleanolic acid | <1000 | Sigma |
| Betulinic acid | <500 | Sigma |
| Rutin | <500 | Sigma |
| Ursolic acid | <500 | Sigma |

As noted in Table 1, compared to other substances of Forsythia suspensa, arctiin and arctigenin are the most effective in melanin synthesis inhibition.

**Experimental Example 2: Melanin synthesis inhibition by arctiin, arctigenin, and the mixture thereof**

An experiment of melanin synthesis inhibition was carried out in the same manner as described in Experimental Example 1, except the following processes. Arctiin, arctigenin, and a 1:3 mixture of arctiin and arctigenin, and a 3:1 mixture of arctiin and arctigenin, which were prepared by Preparation Examples 1 and 2, were diluted in a DMEM medium to final concentrations 60, 100, 140, 180□/□ to treat in the melanoma cells. and then, the cells were further cultured at 37°C for 5 days. Next, the medium was obtained to measure absorbance at 475nm, and melanin synthesis was compared. A melanin synthesis inhibition (%) was calculated according to Mathematical Formula 1, with a control value of cell cultures including only a melanocyte stimulating hormone. Table 2 shows the results.

MathFigure 1

$$\text{Melanin Synthesis Inhibition}(\%) = \left\{ ControlGroupAbsorbance - \frac{TestSampleAbsorbance}{ControlGroupAbsorbance} \right\} X100$$

Table 2

| Melanin synthesis inhibition(%)(n=3) | | |
|---|---|---|
| Tested substances | Final concentration (□/□) | Melanin synthesis inhibition (%) |
| Arctiin | 60 | 67.3 |
| | 100 | 78.5 |
| | 140 | 80.3 |
| | 180 | 84.2 |
| Arctigenin | 60 | 66.8 |
| | 100 | 79.6 |
| | 140 | 81.3 |
| | 180 | 84.3 |
| 1:3 Mixture of Arctiin and Arctigenin | 60 | 80.2 |
| | 100 | 85.7 |
| | 140 | 89.3 |
| | 180 | **90.2** |

(continued)

| Melanin synthesis inhibition(%)(n=3) | | |
|---|---|---|
| Tested substances | Final concentration (□/□) | Melanin synthesis inhibition (%) |
| 3:1 Mixture of Arctiin and Arctigenin | 60 | 66.2 |
| | 100 | 69.3 |
| | 140 | 77.2 |
| | 180 | 80.3 |

As noted in Table 2, melanin synthesis inhibition(%) of arctiin, arctigenin, a 1:3 mixture of arctiin and arctigenin, and a 3:1 mixture of arctiin and arctigenin are increased according to an increase in the concentration. Especially, it is determined that compared to other substances, the 1:3 mixture of arctiin and arctigenin is a very effective.

**Experimental Example 3: Expression inhibition I of a melanin synthesis enzyme in melanocyte**

An experiment was carried out in the same manner as described in Experimental Example 1 except that each of arctiin, arctigenin, matairesinol, oleanolic acid, betulinic acid, rutin, and ursolic acid was added, and culture was further carried out for 3 days to obtain cells. Also, in order to compare the expression inhibition of a melanin synthesis enzyme, experiments on Control Example 1 containing no forsythia suspensa active substance and Control Example 2 containing a melanocyte stimulating hormone were carried out. Obtained cells were washed with sodium phosphate buffer two times, were treated with 0.1% triton X-100, and were reacted by a sonicator for 30 seconds. From the reaction solution, cell precipitate and supernatant were separated by using a high-speed centrifuge at 12,000rpm for 30 minutes at 4°C. From the separated supernatant, protein was separated by using electrophoresis, and was reacted in a reaction solution containing 0.2% DOPA solution for about 8 hours. Then, the production of tyrosinase in gel was measured. Table 3 shows the results.

Table 3

| Inhibiting effect on biosynthesis of tyrosinase (100□/□) | |
|---|---|
| Tested substances | Intensity of tyrosinase band |
| Control. Exp 1 | 123 |
| Control. Exp 2 | **211** |
| Arctiin | 155 |
| Arctigenin | 135 |
| Matairesinol | 180 |
| Oleanolic acid | 200 |
| Betulinic acid | 170 |
| Rutin | 150 |
| Ursolic acid | 180 |

In Table 3, as the intensity of the tyrosinase band is lowered, an inhibiting effect on biosynthesis of tyrosinase is increased. Accordingly, it is determined that arctiin and arctigenin show low band intensities, which is similar to Control Example 1 containing no forsythia suspensa active substance, and show significantly high inhibiting effects compared to Control Example 2 containing only a melanocyte stimulating hormone. Also, the arctiin and arctigenin show higher inhibiting effects on tyrosinase compared to other substances of forsythia suspensa.

**Experimental Example 4: Expression inhibition II of a melanin synthesis enzyme from melanocyte**

An expression inhibiting effect of a melanin synthesis enzyme from melanocyte, which can be achieved by arctiin, arctigenin, or the mixture thereof, was determined. An experiment was carried out in the same manner as described in Experimental Example 1 except that each of arctiin, arctigenin, a 1:3 mixture of arctiin and arctigenin, and

a 3:1 mixture of arctiin and arctigenin prepared by Preparation Examples 1 and 2 was added and culture was further carried out for 3 days to obtain cells. Also, in order to compare the expression inhibition of a melanin synthesis enzyme, experiments on Control Example 1 not containing a melanocyte stimulating hormone and test substance and Control Example 2 containing a melanocyte stimulating hormone were carried out. The obtained cells were washed with sodium phosphate buffer with twice, were treated with 0.1% triton X-100, and were reacted by a sonicator for 30 seconds. From the reaction solution, cell precipitate and supernatant were separated by using a high-speed centrifuge at 12,000rpm for 30 minutes at 4°C. From the separated supernatant, protein was separated by using electrophoresis, and was reacted in a reaction solution containing 0.2% DOPA solution for about 8 hours. And then, the production of tyrosinase was measured in electrophoresis gel. Table 4 shows the results.

Table 4

| Inhibiting effect on biosynthesis of tyrosinase by melanocyte stimulating hormone (□/□) | | | | | | |
|---|---|---|---|---|---|---|
| | Arctiin | Arctigenin | 3:1 Mixture of Arctiin and Arctigenin | 1:3 Mixture of Arctiin and Arctigenin | Melanocyte stimulating hormone | Strength of tyrosinase band |
| Control. Exp 1 | - | - | - | - | 0 | 123 |
| Control. Exp 2 | - | - | - | - | 100 | 211 |
| Exp. 1 | 100 | - | - | - | 100 | 155 |
| Exp. 2 | - | 100 | - | - | 100 | 135 |
| Exp. 3 | - | - | 100 | - | 100 | 145 |
| Exp. 4 | - | - | - | 100 | 100 | 129 |

As noted in Table 4, compared to negative Control Example 1 containing no forsythia suspensa active substance and no melanocyte stimulating hormone, positive Control Example 2 containing a melanocyte stimulating hormone shows the increase of tyrosinase protein. On the other hand, in comparison of the positive Control example, Examples 1~4 containing arctiin, arctiin, arctigenin, a 1:3 mixture of arctiin and arctigenin, and a 3:1 mixture of arctiin and arctigenin show significantly decreased tyrosinase proteins. Especially, the 1:3 mixture of arctiin and arctigenin shows the highest effect. Accordingly, it is determined that arctiin, arctigenin, or the mixture thereof inhibits the expression of a large amount of tyrosinase protein by a melanocyte stimulating hormone.

**Experimental Example 5: Cytotoxicity**

On a fibroblast cell, toxicity of arctiin, arctigenin, a 1:3 mixture of arctiin and arctigenin, and a 3:1 mixture of arctiin and arctigenin prepared by Preparation Examples 1 and 2 was measured, respectively. NIH/3T3(ATCC CRL 1658) cell line (derived from mice) as a fibroblast cell, which was purchased, was inoculated into a DMEM medium including 4.5 g/L of glucose, 10% serum, and 1% antibiotics, and was subcultured in 75□ T-flask at 37°C. The subcultured cell line was cultured under 5% $CO_2$ for 24 hours, and then it was treated with 0.05% Trypsin containing 0.02% EDTA. Then, cells were separated, were inoculated into 96-well-flask ($1 \times 10^4$cells /well) and stabilized through culture for 6 hours. To the cells, each of arctiin, arctigenin, a 1:3 mixture of arctiin and arctigenin, and a 3:1 mixture of arctiin and arctigenin was added as noted in Table 5, and culture was further carried out for 12 hours. After the culture, 10□ of MTT(3-(4,5-dimethylthiazol-2-yl) 2,5-diphenyl tetrazolin bromide) solution (5□/□) was added in each well, and culture was carried out for 4 hours. Then, 100□ of DMSO(Dimethyl sulfoxide) solution was added, and absorbance was measured at O.D. 570nm to calculate the cell viability. Table 5 shows the results based on the criteria as noted in Table 6. Herein, the cell viability of 100% was based on the absorbance in the case of no additional substance.

Table 5

| Viability of fibroblast cell (%) | | |
|---|---|---|
| Tested substances | Final concentration (□/□) | Cell viability (%) |
| Arctiin | 20 | 102.1 |
| | 60 | 101.3 |
| | 100 | 99.8 |
| | 140 | 99.7 |
| | 180 | 99.7 |
| Arctigenin | 20 | 101.2 |
| | 60 | 101.0 |
| | 100 | 99.8 |
| | 140 | 99.6 |
| | 180 | 99.7 |
| 1:3 Mixture of Arctiin and Arctigenin | 20 | 99.8 |
| | 60 | 98.7 |
| | 100 | 94.8 |
| | 140 | 98.3 |
| | 180 | 99.8 |
| 3:1 Mixture of Arctiin and Arctigenin | 20 | 101.1 |
| | 60 | 100.3 |
| | 100 | 99.8 |
| | 140 | 99.9 |
| | 180 | 99.7 |

Table 6

| Criteria of toxicity determination on cells | |
|---|---|
| Range of cell viability | Toxicity determination |
| viability ≤ 70% | Heavily toxic |
| 70% < viability ≤ 80% | Slightly toxic |
| 80% < viability ≤ 90% | Minimally toxic |
| 90% < viability | Non-toxic |

As noted in Table 5, each of the arctiin, arctigenin, and the mixture thereof according to the present invention shows high cell viabilities of 94 to 100%, and thus it is determined that such viability is corresponding to non-toxic, based on the criteria of toxicity determination as noted in Table 6.

**Preparation Example 3: Preparation of cosmetic**

Cosmetics including arctiin prepared by Preparation Example 1 and arctigenin purchased from Sigma as active ingredients were prepared based on the Table 7 (Examples 5, 6, and 7), and also, a cosmetic (Control Example 3) with no additional active substance and a cosmetic (Comparative Example 1) including a conventional whitening substance (that is, arbutin) were prepared.

Table 7

| Content (wt%) | Control Exp. 3 | Exp. 5 | Exp. 6 | Exp. 7 | Comparati ve Exp. 1 |
|---|---|---|---|---|---|
| Arctiin | - | 3.0 | - | 1.5 | - |
| Arctigenin | - | - | 3.0 | 1.5 | - |
| Arbutin | - | - | - | - | 3.0 |
| Glycerin | 5.1 | 5.1 | 5.1 | 5.1 | 5.1 |
| Propylene glycol | 4.2 | 4.2 | 4.2 | 4.2 | 4.2 |
| Tocopheryl acetate | 3.0 | 3.0 | 3.0 | 3.0 | 3.0 |
| Liquid paraffin | 4.6 | 4.6 | 4.6 | 4.6 | 4.6 |
| Triethanolamine | 1.0 | 1.0 | 1.0 | 1.0 | 1.0 |
| Squalene | 3.1 | 3.1 | 3.1 | 3.1 | 3.1 |
| Macadamia nut oil | 2.5 | 2.5 | 2.5 | 2.5 | 2.5 |
| Polysorbate 60 | 1.6 | 1.6 | 1.6 | 1.6 | 1.6 |
| Sorbitan sesquioleate | 1.6 | 1.6 | 1.6 | 1.6 | 1.6 |
| Propylparaben | 0.6 | 0.6 | 0.6 | 0.6 | 0.6 |
| Carboxylic vinyl polymer | 1.5 | 1.5 | 1.5 | 1.5 | 1.5 |
| Fragrance | Minute amount | Minute amount | Minute amount | Minute amount | Minute amount |
| Preservative | Minute amount | Minute amount | Minute amount | Minute amount | Minute amount |
| Purified water | Balance | Balance | Balance | Balance | Balance |
| Total | 100 | 100 | 100 | 100 | 100 |

**Experimental Example 6: Whitening effect of cosmetic composition**

In order to determine the whitening effect of the cosmetic according to the present invention, on cosmetics prepared by Control Example 3, Example 5, Example 6, Example 7, and Comparative Example 1 of Preparation Example 3, a panel test was carried out by 5 groups, the 5 groups randomly including 50 women between the ages of 30 to 40. Each of the cosmetics prepared by Control Example 3, Example 5, Example 6, Example 7, and Comparative Example 1 was applied to panels of each group. In other words, each examinee applied a corresponding cosmetic to her upper arm in an appropriate amount after cleaning every morning and night for 2 months. Before the test, on the skin of each examinee, pigmentation was caused by a UV irradiator. A whitening effect on the upper arm was determined through eye inspection on the skin color. Table 8 shows the results.

Table 8

| Whitening effect of cosmetic | | | | |
|---|---|---|---|---|
| | Effective | Slightly effective | Ineffective | Effectiveness (%) |
| Control Exp. 3 | 3 | 7 | 10 | 15.0 |
| Exp. 5 | 15 | 3 | 2 | 90.0 |
| Exp. 6 | 16 | 2 | 2 | 90.0 |
| Exp. 7 | 18 | 1 | 1 | 95.0 |
| Comparative Exp. 1 | 12 | 3 | 5 | 75.0 |

As noted in Table 8, Example 1 containing arbutin, each of Examples 5 to 7 including arctiin, arctigenin, or 1:1 mixture of arctiin and arctigenin compared to the control Example 3 containing no forsythia suspensia active substance, shows a high whitening effect.

## Experimental Example 7: Skin Irritation

A skin irritation of a cosmetic containing an arctigenin derivative according to the present invention was tested. Herein, as the creams containing arctigenin derivates which was prepared by of Preparation Example 3, Examples 5 to 7, Comparative Example 1, and Control Example 3 were used.

In order to test the skin irritation of a cosmetic, on 50 healthy men and women, a patch test was carried out by using Haye's Test Chamber. A test portion was washed with 70% ethanol and was dried. Then, a patch of 15 □ of test substance or a patch of a Finn chamber (100 × 10, EPITEST, Finland) on which 15 □ of test substance was dropped was tightly attached on the inside portion of forearm of an examinee. After 24 hours, the patch was removed, and the tested portion was marked by a marking pen. After 1 hour and 24 hours after the removal of the patch (that is, after 24 hours and 48 hours from the time the patch was attached), whether erythema or edema occurred or not was determined by observing the tested portion by convex lens (8MC-150, DAZOR, US). The skin reaction was determined based on the criteria of ICDRG (International Contact Dermatitis Research Group) as noted in Table 9, and the mean score of the skin reaction was calculated based on Mathematical Formula 2. Table 10 shows the results.

Table 9

| Determination criteria and grades of skin reaction | | |
|---|---|---|
| Mark | Grade | Determination Criteria |
| - | 0 | No reaction, normal skin |
| ± | 0.5 | Slight or faint erythema |
| + | 1.0 | Clear boundary lined but weak erythema, edema and papule |
| ++ | 2.0 | Clear erythema, papule and small blister |
| +++ | 3.0 | Strong erythema and blister |
| ++++ | 6.0 | Large blister |

MathFigure 2

$$\text{Skin reaction (mean score)} = \sum \frac{\text{reaction} \times \text{number of examinees}}{6(\text{maximum reaction}) \times 20(\text{number of total examinees})} \times \frac{1}{2} \times 100$$

Table 10

| Tested substance | 24 hours | 48 hours | Mean skin reaction (n=20) |
|---|---|---|---|
| | ±+ ++ | ±+ ++ | |
| Control Exp. 3 | 1 -- | 1 -- | 0.42 |
| Exp. 5 | 1 -- | 1 -- | 0.42 |
| Exp. 6 | 1 -- | 1 -- | 0.42 |
| Exp. 7 | 1 -- | 1 -- | 0.42 |
| Comparative Exp. 1 | 1 -- | 1 -- | 0.42 |

As noted in Table 10, a cream of Comparative Example 1, which does not contain an arctigenin derivative according to the present invention, shows very low skin reaction, and thus it is determined that other ingredients of the cream do not cause skin irritation. Also, it is determined that the addition of an arctigenin derivative does not cause skin irritation.

**Experimental Example 8: Formulation Stability**

A formulation stability test according to a change in temperatures was carried out by using cosmetics prepared by Examples 5 to 7, Comparative Example 1, and Control Example 3 of Preparation Example 3. The test samples were charged in an opaque glass vessel and were stored in a thermostat of 45°C for 1 week, or the test samples were charged in an opaque glass vessel and were stored in a completely shaded refrigerator at a constant temperature of 4°C for 1 week. Then, the change in colors was measured by eye inspection. Herein, the change in colors of a product was divided into 6 grades as noted in Table 11. Table 12 shows the results.

Table 11

| Determination criteria on color change of a product | |
|---|---|
| Grade | Determination criteria |
| 0 | Not changed |
| 1 | Extremely slightly changed |
| 2 | Slightly changed |
| 3 | Slightly significantly changed |
| 4 | Significantly changed |
| 5 | Extremely significantly changed |

Table 12

| Formulation stability | | |
|---|---|---|
| | 45°C | 4°C |
| Control Exp. 3 | 0 | 0 |
| Exp. 5 | 0 | 0 |
| Exp. 6 | 0 | 0 |
| Exp. 7 | 0 | 0 |
| Comparative Exp. 1 | 1 | 0 |

As noted in Table 12, it is determined that the temperature stability of a cosmetic composition including arctiin, arctigenin, or the mixture thereof according to the present invention is very excellent. Through the above experiments, it is indirectly determined that cosmetic formation including the arctiin, arctigenin, or the mixture thereof can effectively control or inhibit the synthesis of melanin by acting on a melanin synthesis route of $\alpha$-MSH secreted by UV, thereby whitening the skin color. Hereinafter, Formulation Examples of cosmetics including arctiin, arctigenin, or the mixture thereof will be described. In such Formulation Examples, each cosmetic includes, but is not limited to, arctiin prepared by Preparation Example 1 and arctigenin purchased from Sigma.

**Formulation Examples 1 to 3 : skin softner (Skin lotion)**

From among cosmetics including arctiin or arctigenin as an active ingredient, Formulation Examples of skin softner (skin lotion) are as follows. Form. Exp.1 and 2 are not part of the invention.

Table 13

| Content (wt%) | Form. Exp. 1 | Form. Exp. 2 | Form. Exp. 3 |
|---|---|---|---|
| Arctiin | 1.0 | - | 2.0 |
| Arctigenin | - | 1.0 | 2.0 |
| 1,3-butylene glycol | 5.2 | 5.2 | 5.2 |
| Oleyl alcohol | 1.5 | 1.5 | 1.5 |

(continued)

| Content (wt%) | Form. Exp. 1 | Form. Exp. 2 | Form. Exp. 3 |
|---|---|---|---|
| Ethanol | 3.2 | 3.2 | 3.2 |
| Polysorbate 20 | 3.2 | 3.2 | 3.2 |
| Benzophenone-9 | 2.0 | 2.0 | 2.0 |
| Carboxylic vinyl polymer | 1.0 | 1.0 | 1.0 |
| Glycerin | 3.5 | 3.5 | 3.5 |
| Fragrance | Minute amount | Minute amount | Minute amount |
| Preservative | Minute amount | Minute amount | Minute amount |
| Purified water | Balance | Balance | Balance |
| Total | 100 | 100 | 100 |

**Formulation Examples 4 to 6 : Nutrition Lotion (Milk Lotion**)

From among cosmetics including arctiin or arctigenin, Formulation Examples of nutrition lotion (milk lotion) are as follows. Form. Exp. 4 and 5 are not part of the invention.

Table 14

| Content (wt%) | Form. Exp. 4 | Form. Exp. 5 | Form. Exp. 6 |
|---|---|---|---|
| Arctiin | 2.5 | - | 1 |
| Arctigenin | - | 1.5 | 7 |
| Glycerin | 5.1 | 5.1 | 5.1 |
| Propylene glycol | 4.2 | 4.2 | 4.2 |
| Tocopheryl acetate | 3.0 | 3.0 | 3.0 |
| Liquid paraffin | 4.6 | 4.6 | 4.6 |
| Triethanolamine | 1.0 | 1.0 | 1.0 |
| Squalene | 3.1 | 3.1 | 3.1 |
| Macadamia nut oil | 2.5 | 2.5 | 2.5 |
| Polysorbate 60 | 1.6 | 1.6 | 1.6 |
| Sorbitan sesquioleate | 1.6 | 1.6 | 1.6 |
| Propylparaben | 0.6 | 0.6 | 0.6 |
| Carboxylic vinyl polymer | 1.5 | 1.5 | 1.5 |
| Fragrance | Minute amount | Minute amount | Minute amount |
| Preservative | Minute amount | Minute amount | Minute amount |
| Purified water | Balance | Balance | Balance |
| Total | 100 | 100 | 100 |

**Formulation Examples 7 to 9 : Nutrition Cream**

From among cosmetics including arctiin or arctigenin, Formulation Examples of nutrition cream are as follows.

Table 15

| Content (wt%) | Form. Exp. 7 | Form. Exp. 8 | Form. Exp. 9 |
|---|---|---|---|
| Arctiin | 1 | - | 3 |
| Arctigenin | - | 7 | 3 |
| Glycerin | 4.0 | 4.0 | 4.0 |
| Vaseline | 3.5 | 3.5 | 3.5 |
| Triethanolamine | 2.1 | 2.1 | 2.1 |
| Liquid paraffin | 5.3 | 5.3 | 5.3 |
| Squalene | 3.0 | 3.0 | 3.0 |
| Beeswax | 2.6 | 2.6 | 2.6 |
| Tocopheryl acetate | 5.4 | 5.4 | 5.4 |
| Polysorbate 60 | 3.2 | 3.2 | 3.2 |
| Carboxylic vinyl polymer | 1.0 | 1.0 | 1.0 |
| Sorbitan sesquioleate | 3.1 | 3.1 | 3.1 |
| Fragrance | Minute amount | Minute amount | Minute amount |
| Preservative | Minute amount | Minute amount | Minute amount |
| Purified water | Balance | Balance | Balance |
| Total | 100 | 100 | 100 |

**Formulation Examples 10 to 12 : Massage Cream**

From among cosmetics including arctiin or arctigenin, Formulation Examples of massage cream are as follows. Form. Exp. 10 and 11 are not part of the invention.

Table 16

| Content (wt%) | Form. Exp. 10 | Form. Exp. 11 | Form. Exp. 12 |
|---|---|---|---|
| Arctiin | 3 | - | 3 |
| Arctigenin | - | 5 | 5 |
| Glycerin | 4.0 | 4.0 | 4.0 |
| Vaseline | 3.5 | 3.5 | 3.5 |
| Triethanolamine | 0.5 | 0.5 | 0.5 |
| Liquid paraffin | 24.0 | 24.0 | 24.0 |
| Squalene | 3.0 | 3.0 | 3.0 |
| Beeswax | 2.1 | 2.1 | 2.1 |
| Tocopheryl acetate | 0.1 | 0.1 | 0.1 |
| Polysorbate 60 | 2.4 | 2.4 | 2.4 |
| Carboxylic vinyl polymer | 1.0 | 1.0 | 1.0 |
| Sorbitan sesquioleate | 2.3 | 2.3 | 2.3 |
| Fragrance | Minute amount | Minute amount | Minute amount |
| Preservative | Minute amount | Minute amount | Minute amount |
| Purified water | Balance | Balance | Balance |
| Total | 100 | 100 | 100 |

**Formulation Examples 13 to 15 : Pack**

From among cosmetics including arctiin or arctigenin, Formulation Examples of pack are as follows. Form. Exp. 13 and 14 are not part of the invention.

Table 17

| Content (wt%) | Form. Exp. 13 | Form. Exp. 14 | Form. Exp. 15 |
|---|---|---|---|
| Arctiin | 2 | - | 3 |
| Arctigenin | - | 5 | 3 |
| Ethyl alcohol | 3.0 | 3.0 | 3.0 |
| EDTA-2Na | 0.02 | 0.02 | 0.02 |
| Propylene glycol | 5.1 | 5.1 | 5.1 |
| Glycerin | 4.5 | 4.5 | 4.5 |
| Carbopol | 1.0 | 1.0 | 1.0 |
| Poly oxide | 0.1 | 0.1 | 0.1 |
| Preservative | Minute amount | Minute amount | Minute amount |
| Fragrance | Minute amount | Minute amount | Minute amount |
| Purified water | Balance | Balance | Balance |
| Total | 100 | 100 | 100 |

**Advantageous Effects**

The present invention provides a skin whitening cosmetic composition comprising arctiin, arctigenin or the mixture thereof as an active ingredient. Forsythia suspensa ingredients used for the cosmetic in the present invention, such as arctiin, arctigenin or the mixture thereof, can control and inhibit the synthesis of melanin by acting on a melanin synthesis mechanism induced by $\alpha$-MSH, and show a excellent whitening effect and lower skin irritation, compared to a conventional skin whitening cosmetic.

While this invention has been described in connection with what is presently considered to be the most practical and exemplary embodiment, it is to be understood that the invention is not limited to the disclosed embodiment and the drawings, but, on the contrary, it is intended to cover various modifications and variations within the spirit and scope of the appended claims.

**Claims**

1. A use of a cosmetic composition for skin whitening, wherein the cosmetic composition comprises a mixture of arctiin and arctigenin as an active ingredient, said mixture of arctiin and arctigenin has a weight ratio of 3:1 to 1:3 and is included in an amount ranging from 0.001 to 15.0 wt %, based on a total weight of the cosmetic composition to inhibit melanin synthesis.

2. The use of a cosmetic composition for skin whitening as claimed in claim 1, wherein a formulation of the composition selected from the group including solution, suspension, emulsion, paste, gel, cream, lotion, powder, soap, surfactant containing cleaning, oil, powder foundation, emulsion foundation, wax foundation, and spray.

3. The use of a cosmetic composition for skin whitening according to claim 1 and 2, wherein, in the mixture, the arctiin and the arctigenin are mixed in a weight ratio of 1:3.

4. The use of a cosmetic composition for skin whitening according to any one of the preceding claims, wherein said mixture of arctiin and arctigenin is included in an amount ranging from 0.1 to 8.0 wt %, based on a total weight of the cosmetic composition.

5. The use of a cosmetic composition for skin whitening according to any one of the preceding claims, wherein said cosmetic composition comprises additives selected from: an antioxidant, a stabilizer, a solubilizing agent, vitamins, a pigment and perfume, carrier components, or mixture thereof.

6. The use of a cosmetic composition for skin whitening according to claim 2, wherein when the formulation of the composition is selected from a paste, a cream or a gel, said formulation comprises a carrier ingredient composed of animal oil, vegetable oil, wax, paraffin, starch, tragacanth, a cellulose derivative, polyethylene glycol, silicon, bentonite, silica, talc, zinc oxide, or mixture thereof.

7. The use of a cosmetic composition for skin whitening according to claim 2, wherein when the formulation of the composition is selected from a powder or a spray, said formulation comprises a carrier ingredient composed of lactose, talc, silica, aluminum hydroxide, calcium silicate, polyamide powder, or mixture thereof.

8. The use of a cosmetic composition for skin whitening according to claim 2, wherein when the formulation of the composition is selected from a solution or an emulsion, said formulation comprises a carrier ingredient composed of a solvent, a solubilizer or an emulsifier, or mixture thereof.

9. The use of a cosmetic composition for skin whitening according to claim 2, wherein when the formulation of the composition is selected from a suspension, said formulation comprises a carrier ingredient composed of a liquid diluent, a suspension, microcrystalline cellulose, aluminum metahydroxide, bentonite, agar, tragacanth, or mixture thereof.

10. The use of a cosmetic composition for skin whitening according to claim 2, wherein when the formulation of the composition is selected from a surfactant containing cleansing, said formulation comprises a carrier ingredient composed of aliphatic alcohol sulfate, aliphatic alcohol ether sulfate, sulfosuccinic acid monoester, isethionate, an imidazolinium derivative, methyl taurate, sarcosinate, fatty acid amide ether sulfate, alkyl amido betaine, aliphatic alcohol, fatty acid glyceride, fatty acid diethanolamide, vegetable oil, lanolin derivative, ethoxylated glycerol fatty acid ester, or mixture thereof.

11. The use of a cosmetic composition for skin whitening according to any one of the preceding claims, wherein said mixture of arctiin and arctigenin inhibits the synthesis of melanin by acting on a melanin synthesis route of $\alpha$-melanocyte stimulating hormone ($\alpha$-MSH).

## Patentansprüche

1. Verwendung einer kosmetischen Zusammensetzung zur Hautbleichung, wobei die kosmetische Zusammensetzung eine Mischung aus Arctiin und Arctigenin als Wirkstoff enthält, die Mischung aus Arctiin und Arctigenin ein Gewichtsverhältnis von 3:1 bis 1:3 aufweist und in einer Menge im Bereich von 0,001 bis 15 Gew.-%, bezogen auf das Gesamtgewicht der kosmetischen Zusammensetzung, enthalten ist, um die Melaninsynthese zu hemmen.

2. Verwendung einer kosmetischen Zusammensetzung zur Hautbleichung nach Anspruch 1, wobei eine Formulierung der Zusammensetzung aus der Gruppe ausgewählt ist, die Lösung, Suspension, Emulsion, Paste, Gel, Creme, Lotion, Puder, Seife, tensidhaltige Reinigung, Öl, Puder-Grundierung, Emulsionsgrundierung, Wachsgrundierung, und Spray umfasst.

3. Verwendung einer kosmetischen Zusammensetzung zur Hautbleichung nach Anspruch 1 und 2, wobei das Arctiin und das Arctigenin in der Mischung in einem Gewichtsverhältnis von 1:3 gemischt sind.

4. Verwendung einer kosmetischen Zusammensetzung zur Hautbleichung nach einem der vorhergehenden Ansprüche, wobei die Mischung aus Arctiin und Arctigenin in einer Menge im Bereich von 0,1 bis 8,0 Gew.-%, bezogen auf das Gesamtgewicht der kosmetischen Zusammensetzung, enthalten ist.

5. Verwendung einer kosmetischen Zusammensetzung zur Hautbleichung nach einem der vorhergehenden Ansprüche, wobei die kosmetische Zusammensetzung Zusatzstoffe enthält, die ausgewählt sind aus: einem Antioxidans, einem Stabilisierungsmittel, einem Lösungsvermittler, Vitaminen, einem Pigment und einem Parfüm, Trägerbestandteilen, oder einer Mischung davon.

6. Verwendung einer kosmetischen Zusammensetzung zur Hautbleichung nach Anspruch 2, wobei, wenn die Formulierung der Zusammensetzung aus einer Paste, einer Creme oder einem Gel ausgewählt ist, die Formulierung einen Trägerstoff enthält, der aus tierischem Öl, pflanzlichem Öl, Wachs, Paraffin, Stärke, Traganth, einem Cellulosederivat, Polyethylenglykol, Silikon, Bentonit, Kieselerde, Talkum, Zinkoxid, oder einer Mischung davon ausgewählt ist.

7. Verwendung einer kosmetischen Zusammensetzung zur Hautbleichung nach Anspruch 2, wobei, wenn die Formulierung der Zusammensetzung aus einem Puder oder einem Spray ausgewählt ist, die Formulierung einen Trägerstoff umfasst, der aus Laktose, Talkum, Kieselerde, Aluminiumhydroxid, Calciumsilikat, Polyamidpulver, oder einer Mischung davon besteht.

8. Verwendung einer kosmetischen Zusammensetzung zur Hautbleichung nach Anspruch 2, wobei, wenn die Formulierung der Zusammensetzung aus einer Lösung oder einer Emulsion ausgewählt ist, die Formulierung einen Trägerstoff enthält, der aus einem Lösungsmittel, einem Lösungsvermittler oder einem Emulgator, oder einer Mischung davon besteht.

9. Verwendung einer kosmetischen Zusammensetzung zur Hautbleichung nach Anspruch 2, wobei, wenn die Formulierung der Zusammensetzung aus einer Suspension ausgewählt ist, die Formulierung einen Trägerstoff enthält, der aus einem flüssigen Verdünnungsmittel, einer Suspension, mikrokristalliner Cellulose, Aluminiummetahydroxid, Bentonit, Agar, Traganth, oder einer Mischung davon besteht.

10. Verwendung einer kosmetischen Zusammensetzung zur Hautbleichung nach Anspruch 2, wobei, wenn die Formulierung der Zusammensetzung aus einem tensidhaltigen Reinigungsmittel ausgewählt ist, die Formulierung einen Trägerstoff enthält, der aus aliphatischem Alkoholsulfat, aliphatischem Alkoholethersulfat, Sulfonbernsteinsäuremonoester, Isethionat, einem Imidazoliniumderivat, Methyltaurat, Sarkosinat, Fettsäureethersulfat, Alkylamidobetain, aliphatischem Alkohol, Fettsäureglycerid, Fettsäurediethanolamid, pflanzlichem Öl, Lanolinderivat, ethoxyliertem Glycerinfettsäureester, oder einer Mischung davon besteht.

11. Verwendung einer kosmetischen Zusammensetzung zur Hautbleichung nach einem der vorhergehenden Ansprüche, wobei die Mischung aus Arctiin und Arctigenin die Synthese von Melanin hemmt, indem sie auf einen Melaninsyntheseweg von $\alpha$-Melanozyten stimulierendem Hormon ($\alpha$-MSH) wirkt.

**Revendications**

1. Utilisation d'une composition cosmétique pour le blanchiment de la peau, où la composition cosmétique comprend un mélange d'arctiine et d'arctigénine en tant qu'ingrédient actif, ledit mélange d'arctiine et d'arctigénine a un rapport pondéral de 3:1 à 1:3 et est incorporé en une quantité allant de 0,001 à 15,0% en poids, basée sur le poids total de la composition cosmétique, pour inhiber la synthèse de mélanine.

2. Utilisation d'une composition cosmétique pour le blanchiment de la peau telle que revendiquée dans la revendication 1, une formulation de la composition étant choisie dans le groupe comprenant une solution, une suspension, une émulsion, une pâte, un gel, une crème, une lotion, une poudre, un savon, un nettoyant contenant du tensioactif, une huile, un fond de teint poudre, un fond de teint en émulsion, un fond de teint à la cire, et un spray.

3. Utilisation d'une composition cosmétique pour le blanchiment de la peau selon les revendications 1 et 2, où, dans le mélange, l'arctiine et l'arctigénine sont mélangées dans un rapport pondéral de 1:3.

4. Utilisation d'une composition cosmétique pour le blanchiment de la peau selon l'une quelconque des revendications précédentes, où ledit mélange d'arctiine et d'arctigénine est incorporé en une quantité allant de 0,1 à 8,0% en poids, basée sur le poids total de la composition cosmétique.

5. Utilisation d'une composition cosmétique pour le blanchiment de la peau selon l'une quelconque des revendications précédentes, où ladite composition cosmétique comprend des additifs choisis parmi : un antioxydant, un stabilisant, un agent solubilisant, des vitamines, un pigment et un parfum, des composants supports, ou un de leurs mélanges.

6. Utilisation d'une composition cosmétique pour le blanchiment de la peau selon la revendication 2, où lorsque la formulation de la composition est choisie parmi une pâte, une crème ou un gel, ladite formulation comprend un ingrédient support composé d'huile animale, d'huile végétale, de cire, de paraffine, d'amidon, d'adragante, d'un

dérivé de cellulose, de polyéthylèneglycol, de silicium, de bentonite, de silice, de talc, d'oxyde de zinc, ou d'un de leurs mélanges.

**7.** Utilisation d'une composition cosmétique pour le blanchiment de la peau selon la revendication 2, où lorsque la formulation de la composition est choisie parmi une poudre ou un spray, ladite formulation comprend un ingrédient support composé de lactose, de talc, de silice, d'hydroxyde d'aluminium, de silicate de calcium, de poudre de polyamide, ou d'un de leurs mélanges.

**8.** Utilisation d'une composition cosmétique pour le blanchiment de la peau selon la revendication 2, où lorsque la formulation de la composition est choisie parmi une solution ou une émulsion, ladite formulation comprend un ingrédient support composé d'un solvant, d'un solubilisant ou d'un émulsifiant, ou d'un de leurs mélanges.

**9.** Utilisation d'une composition cosmétique pour le blanchiment de la peau selon la revendication 2, où lorsque la formulation de la composition est choisie parmi une suspension, ladite formulation comprend un ingrédient support composé d'un diluant liquide, d'une suspension, de cellulose microcristalline, de métahydroxyde d'aluminium, de bentonite, de gélose, d'adragante, ou d'un de leurs mélanges.

**10.** Utilisation d'une composition cosmétique pour le blanchiment de la peau selon la revendication 2, où lorsque la formulation de la composition est choisie parmi un nettoyant contenant du tensioactif, ladite formulation comprend un ingrédient support composé de sulfate d'alcool aliphatique, d'éther sulfate d'alcool aliphatique, de monoester d'acide sulfosuccinique, d'iséthionate, d'un dérivé d'imidazolinium, de taurate de méthyle, de sarcosinate, d'éther sulfate d'amide d'acide gras, d'alkylamidobétaïne, d'alcool aliphatique, de glycéride d'acide gras, de diéthanolamide d'acide gras, d'huile végétale, de dérivé de lanoline, d'ester d'acide gras de glycérol éthoxylé, ou d'un de leurs mélanges.

**11.** Utilisation d'une composition cosmétique pour le blanchiment de la peau selon l'une quelconque des revendications précédentes, où ledit mélange d'arctiine et d'arctigénine inhibe la synthèse de mélanine en agissant sur une voie de synthèse de mélanine de l'hormone mélanotrope $\alpha$ ($\alpha$-MSH).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **ANDERSON JJB ; GARNER SC.** Nutr. Res. The Royal Soc. of Chemistry, 1996, vol. 17, 1617-1632